(19) Europäisches Patentamt
European Patent Office
Office européen des brevets

(11) **EP 2 019 088 B1**

(12) **EUROPÄISCHE PATENTSCHRIFT**

(45) Veröffentlichungstag und Bekanntmachung des Hinweises auf die Patenterteilung:
**20.06.2012 Patentblatt 2012/25**

(21) Anmeldenummer: **08160167.6**

(22) Anmeldetag: **11.07.2008**

(51) Int Cl.:
*C07C 29/74* (2006.01)     *C07C 67/03* (2006.01)
*C10L 1/02* (2006.01)     *B01D 15/36* (2006.01)
*B01J 47/04* (2006.01)     *C07C 29/76* (2006.01)
*C11C 3/00* (2006.01)     *C11C 3/10* (2006.01)

(54) **Glycerinreinigung**

Glycerol cleaning

Nettoyage de glycérol

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **25.07.2007 DE 102007034621**

(43) Veröffentlichungstag der Anmeldung:
**28.01.2009 Patentblatt 2009/05**

(73) Patentinhaber: **LANXESS Deutschland GmbH 51369 Leverkusen (DE)**

(72) Erfinder:
- **Soest, Hans-Karl**
  **51147, Köln (DE)**
- **Litzinger, Ulrich**
  **57627, Hachenburg (DE)**
- **Klipper, Reinhold**
  **50933, Köln (DE)**
- **Wagner, Rudolf**
  **51061, Köln (DE)**

(56) Entgegenhaltungen:
**EP-A- 0 411 780      EP-A- 1 075 795**
**WO-A-89/05861      US-A- 5 177 009**
**US-A1- 2006 014 974**

- **HARRIS ET AL: "Glycerol Production: A Pilot-Plant Investigation for Continuous Fermentation and Recovery" JOURNAL OF BIOCHEMICAL AND MICROBIOLOGICAL TECHNOLOGY AND ENGINEERING, Bd. II, Nr. 1, 1960, Seiten 9-24, XP002504185**
- **Dow Water Solutions DOWEX TM Fine Mesh Spherical Ion Exchnage Resins**
- **IonXchange LLC Product List**
- **Produktinformation LEWATIT S2528**

Bemerkungen:
Die Akte enthält technische Angaben, die nach dem Eingang der Anmeldung eingereicht wurden und die nicht in dieser Patentschrift enthalten sind.

EP 2 019 088 B1

**Beschreibung**

Gegenstand der vorliegenden Erfindung ist ein Verfahren zur Reinigung von Glycerin, mittels monodisperser Ionenaustauscher in einer Reinigungseinheit aus Elektrolytvorlaufverfahren und einem Mischbett wobei sowohl im Elektrolytvorlaufverfahren als auch im Mischbett jeweils wenigstens ein monodisperser Ionenaustauscher eingesetzt wird und diese aus monodispersen Perlpolymerisaten durch Verdüsungs- oder Saatzulaufverfahren hergestellt werden, sowie eine Apparatur zur Biodieselherstellung die eine solche Reinigungseinheit enthält.

Durch die in jüngster Zeit verstärkte Synthese von Biodiesel aus nachwachsenden Rohstoffen fallen erhebliche Mengen Glycerin an, das prozessbedingt erheblich mit Ionen, insbesondere Natrium und Chlorid, belastet ist sowie tiefbraune Verfärbungen aufweisen kann. Beides ist jedoch für die weitere Verarbeitung von Glycerin, beispielsweise in Kosmetika, in der Lebensmittelindustrie oder in pharmazeutischen Produkten, unerwünscht.

Glycerin mittels Ionenaustauscher zu reinigen ist bekannter Stand der Technik. US 7, 126,032 B1 beschreibt die Reinigung von Glycerin aus der Biodiesel Fabrikation unter anderem mit Ionenaustauschern. Ebenso empfehlen zahlreiche Produktbroschüren namhafter Hersteller von Ionenaustauschern den Einsatz von Ionenaustauschern zur Entsalzung von Glycerin, beispielsweise die Dow Chemical Company Dowex® HCR-W2, einen starksauren, gelförmigen Kationenaustauscher (16 - 40 mesh), oder die Lanxess Deutschland GmbH unter der Marke Lewatit® die Ionenaustauscher S1428, S1468, S2528, S2568, S3428, S4228, S4268, S4328, S6328, S6368 oder MDS1368Natrium.

Harris et al, Glycerol Production, Journal of Biochemical and Microbiological Technology and Engineering, Bd. II, Nr. 1, 1960, 9-24 offenbart ein Verfahren und eine Apparatur zur Reinigung von Glycerin mit Hilfe einer Kombination von Elektrolytvorlaufverfahren und Mischbett (Abbildung 2, S. 20-22).

EP-A 0 411 780 offenbart ein Verfahren und eine Apparatur zur Reinigung von Glycerin mit Hilfe einer Kombination von Elektrolytvorlaufverfahren und Mischbett (Beispiel 5, D2; Beispiel 2).

US 2006/014 974 A1 offenbart ein Verfahren zur Herstellung von Biodiesel umfassend Transesterifizierung, Trennung des Esters vom Polyol (Glycerin), Reinigung des Glycerins mit Hilfe eines Vakuumverdampfers und Reinigung des Esters mit Hilfe von Ionenaustauschern.

WO 89/05861 A beschreibt ein Verfahren zur Herstellung von Ethanol und zur Gewinnung von Glycerin als Nebenprodukt desselben, das die folgenden Stufen umfasst:

Herstellung einer Biomassen-Maische;

Fermentation der Maische mit Hefe;

Destillation der fermentierten Maische unter Bildung von Ethanol und Glycerin enthaltender Schlempe;

Klärung der Schlempe; und

Hindurchleiten der geklärten Schlempe durch ein Ionenausschlussmaterial zur Abtrennung des Glycerins von den anderen Bestandteilen der geklärten Schlempe,

das dadurch gekennzeichnet ist, dass die Schlempe geklärt wird, indem man sie einem Querstrom(Kreuzstrom)-Mikrofiltrationssystem aussetzt, das Keramik- oder Mineral-Membranen aufweist und in der Lage ist, Teilchen im Größenbereich von 0,1 bis 10 $\mu$m abzutrennen.

Nicht immer werden durch den Einsatz der genannten Ionenaustauscher die für bestimmte Industriezweige erforderlichen Reinheiten des Glycerins erreicht. Es besteht daher das Bestreben, Glycerin in einer solchen Reinheit zu erhalten, dass dieses den hohen Anforderungen der Kosmetikindustrie, der Lebensmittelindustrie oder der pharmazeutischen Industrie an ihre Rohstoffe erfüllt.

Lösung der Aufgabe und somit Gegenstand der vorliegenden Erfindung ist ein Verfahren zur Reinigung von Polyolen dadurch gekennzeichnet, dass man eine Reinigungseinheit aus Elektrolytvorlaufverfahren und einem Mischbett einsetzt., wobei sowohl im Elektrolytvorlaufverfahren als auch im Mischbett jeweils wenigstens ein monodisperser Ionenaustauscher eingesetzt wird und diese aus monodispersen Perlpolymerisaten durch Verdüsungs- oder Saatzulaufverfahren hergestellt werden.

Das Elektrolytvorlaufverfahren, im Folgenden als IEC (englisch: Ion Exclusion Chromatography) bezeichnet, ist bekannter Stand der Technik. Auch als Ionenausschluss-Chromatographie bezeichnet, wird es beispielsweise zur Fraktionierung von Silagesaft in eine Aminosäuren- und eine Milchsäurefraktion eingesetzt.

In der älteren deutschsprachigen Literatur wird für die Ionenausschluss-Chromatographie der gebräuchlichere Name Elektrolytvorlaufverfahren benutzt. Dieser beschreibt den primären Trennmechanismus sehr gut. Elektrolyte (anorganische Ionen, organische Ionen) werden aus der Ionentauschermatrix ausgeschlossen und die gesamte Elektrolytfraktion passiert die Chromatographiesäule als wäre diese mit Glaskugeln befüllt. Beispielsweise wird die IEC auch zur Abtrennung von Zuckern (WO 2003056038 A1) oder in der Ethanolgewinnung (WO 1995017517 A1) eingesetzt.

Zum Einsatz in der IEC werden zahlreiche Ionenaustauscher, so auch monodisperse Ionenaustauscher bereitgehalten. So finden sich unter http://www.dow.com/liquidseps/prod/chromato.htm das monodisperse Dowex ® Monosphere 99K 320 zum Einsatz in der Aminosäurenproduktion oder der Produktion organischer Säuren sowie zur Herstellung von Zucker aus Zuckerrüben oder Zuckerrohr. In der vorliegenden Erfindung wird die IEC als Verfahren zur Entsalzung des Polyols, bevorzugt des Glycerins genutzt.

Ein Mischbett, bzw. Mischbett-Harze, sind eine Mischung aus zumeist einem stark sauren Kationenaustauscher und einem stark basischen Anionenaustauscher, die optimal aufeinander abgestimmt sind. Ohne Probleme entfernen diese Harze auch "schwierige" Wasserinhaltsstoffe, wie z.B. Kieselsäure und Kohlensäure. Sie werden bevorzugt zur Vollentsalzung von Wasser eingesetzt. Beispielhaft werden Mischbetten in US 2005/0103622 A1 und insbesondere in US - A 5,858,191 beschrieben, insbesondere letztere wird diesbezüglich von der vorliegenden Anmeldung vollumfänglich umfasst.

Im Gegensatz zu heterodispersen Ionenaustauschern mit heterodisperser Teilchengrößenverteilung, die man durch traditionelle Herstellweisen erhält, werden in der vorliegenden Anmeldung als monodispers solche Ionenaustauscher bezeichnet, bei denen mindestens 90 Volumen- oder Massen-% der Teilchen einen Durchmesser besitzen, der in dem Intervall mit der Breite von ± 10 % des häufigsten Durchmesser um den häufigsten Durchmesser herum liegt.

Zum Beispiel bei einem Ionenaustauscher mit häufigstem Perldurchmesser von 0,5 mm liegen mindestens 90-Volumen- oder Massen-% in einem Größenintervall zwischen 0,45 mm und 0,55 mm, bei einem Stoff mit häufigstem Durchmesser von 0,7 mm liegen mindestens 90 Volumen- oder Massen-% in einem Größenintervall zwischen 0,77 mm und 0,63 mm.

Ein für die Herstellung monodisperser Ionenaustauscher erforderliches monodisperses Perlpolymerisat kann nach den aus der Literatur bekannten Verfahren hergestellt werden. Beispielsweise werden solche Verfahren und daraus herzustellende monodisperse Ionenaustauscher in US-A 4 444 961, EP-A 0 046 535, US 4 419 245 oder WO 93/12167 beschrieben, deren Inhalte von der vorliegenden Anmeldung vollumfänglich mit umfasst werden. Erfindungsgemäß werden monodisperse Perlpolymerisate und die daraus herzustellenden monodispersen Ionenaustauscher durch Verdüsungs-(Jetting) oder Saatzulaufverfahren (seed/feed Prozess) erhalten. Erfindungsgemäß sind sowohl im IEC als auch im Mischbett jeweils ein monodisperser Ionenaustauscher enthalten, die durch Verdüsungs-(Jetting) oder Saatzulaufverfahren (seed/feed Prozess) erzeugt wurden. Erfindungsgemäß ganz besonders bevorzugt sind sowohl im IEC als auch im Mischbett nur monodisperse Ionenaustauscher enthalten.

Erfindungsgemäß bevorzugt werden im IEC starksaure Kationenaustauscher , besonders bevorzugt starksaure, gelförmige Kationer eingesetzt. Erfindungsgemäß insbesondere bevorzugt werden im IEC monodisperse, starksaure, gelförmige Kationer, beispielsweise Lewatit® GF 303, eingesetzt.

Das nach der Behandlung im IEC erhaltene weitestgehend salzfreie Glycerin, wird erfindungsgemäß in der zweiten Stufe einer Feinreinigung in einem Mischbett unterzogen, wodurch ein Salzgehalt von unter 1 ppm angestrebt wird. Zusätzlich erzielt man im Mischbett ein sogenanntes Polishing des Glycerins wodurch ein sehr niedriger Farbwert bis nahezu zur vollständigen Kläre erzielt wird. Hierzu werden bevorzugt ein Anionenaustauscher und ein Kationenaustauscher nebeneinander eingesetzt. Eines der im Mischbett einzusetzenden Harze ist monodispers, insbesondere bevorzugt sind im Mischbett beide Ionenaustauscher monodispers.

Die Begriffe mikroporös bzw. makroporös oder gelförmig sind in der Fachliteratur bereits eingehend beschrieben worden. Bevorzugte Anionenaustauscher bzw. Kationenaustauscher im Mischbett weisen eine makroporöse Struktur auf.

Die Ausbildung makroporöser Perlpolymerisate zur Herstellung makroporöser Ionenaustauscher kann beispielsweise durch Zusatz von Inertmaterialien (Porogene) zu der Monomermischung bei der Polymerisation erfolgen. Als solche sind vor allem organische Substanzen geeignet, die sich im Monomeren lösen, das Polymerisat aber schlecht lösen bzw. quellen (Fällmittel für Polymere) beispielsweise aliphatische Kohlenwasserstoffe (Farbenfabriken Bayer DBP 1045102, 1957; DBP 1113570, 1957).

In US-A 4,382,124 werden als Porogen beispielsweise Alkohole mit 4 bis 10 Kohlenstoffatomen zur Herstellung monodisperser, makroporöser Perlpolymerisate auf Styrol/Divinylbenzol-Basis eingesetzt. Ferner wird eine Übersicht der Herstellmethoden makroporöser Perlpolymerisate gegeben. Erfindungsgemäß bevorzugt sind als Porogene organische Lösungsmittel geeignet, die das entstandene Polymerisat schlecht lösen bzw. quellen. Bevorzugte Porogene sind Hexan, Octan, Isooctan, Isododecan, Methylethylketon, Butanol oder Octanol oder deren Isomeren. Erfindungsgemäß bevorzugt ist daher im Mischbett die Kombination eines monodispersen, makroporösen Kationenaustauschers mit einem monodispersen, makroporösen Anionenaustauscher, besonders bevorzugt einem monodispersen, makroporösen, starksauren Kationer mit einem monodispersen, makroporösen, mittelbasischen Anioner. Als Beispiel für ein Mischbett sei an dieser Stelle Lewatit® GF 404 in Kombination mit Lewatit® GF 505 genannt.

Überraschenderweise erzielt man durch das erfindungsgemäße Verfahren, nämlich mittels einer Reinigungseinheit aus IEC und einem Mischbett, Glycerin in solch hoher Reinheit und solch hervorragenden Farbzahlen, dass dieses ohne weitere Aufarbeitung in der Kosmetikindustrie, der Lebensmittelindustrie oder pharmazeutischen Industrie eingesetzt werden können. Im Falle des Glycerins werden Salzgehalte von weniger als 1 ppm und Farbwerte von weniger als 1 IU (International Unit = internationale Einheit; Sugar Analysis, Icumsa Methods, F.Schneider, 1979, Paragraph 7, Physical Characteristics of Colour of Sugar and Solutions) erzielt.

Die vorliegende Erfindung betrifft aber auch die Verwendung wenigstens eines, bevorzugt wenigstens zwei, besonders bevorzugt wenigstens drei monodisperser Ionenaustauscher innerhalb einer Reinigungseinheit aus IEC und Mischbett zur Reinigung von Glycerin.

Die vorliegende Erfindung betrifft ferner die Verwendung einer Reinigungseinheit aus IEC und Mischbett bei der Herstellung von Biodiesel zur Aufarbeitung des bei der Produktion anfallenden Glycerins. Gegenstand der vorliegenden Erfindung ist deshalb auch eine Apparatur zur Biodieselherstellung enthaltend eine Reinigungseinheit aus wenigstens einem Elektrolytvorlaufverfahren und wenigstens einem Mischbett zur Glycerinreinigung. Die Erfindung betrifft ferner ein Verfahren zur Herstellung von Biodiesel, dadurch gekennzeichnet, dass der Stoffstrom des Glycerins einer Reinigungseinheit aus IEC und einem Mischbett unterzogen wird. In einer bevorzugten Ausführungsform ist das Verfahren gekennzeichnet, durch

a) die Transesterifizierung freier Fettsäuren zu Fettsäureestern mittels makroporöser Kationer,

b) die Trennung des Biodiesels vom Glycerin und

c1) die Aufarbeitung des Glycerins mittels einer Reinigungseinheit aus IEC und Mischbett sowie

c2) die Aufarbeitung des Biodiesels zur Entfernung von Glycerin und/oder Seifen durch einen starksauren, monodispersen, makroporösen Kationer.

In einer besonders bevorzugten Ausführungsform wird im Schritt a) ein heterodisperser, makroporöser, hochsulfonierter Kationenaustauscher und im Schritt c2) ein starksaurer, monodisperser, gelförmiger Kationenaustauscher eingesetzt.

Die erfindungsgemäße Apparatur zur Biodieselherstellung enthaltend eine Reinigungseinheit aus IEC und Mischbett zur Aufarbeitung des bei der Produktion von Biodiesel anfallenden Glycerins, kann zusätzlich zu dem oder den monodispersen Ionenaustauscher(n) in der Reinigungseinheit mit zusätzlichen monodispersen Ionenaustauschern in der Biodiesel Aufarbeitung selber versehen sein. In einer bevorzugten Ausführungsform ist die Apparatur zur Herstellung von Biodiesel gekennzeichnet durch die in einzelnen Apparaten durchzuführenden Arbeitsschritte

a) die Transesterifizierung freier Fettsäuren zu Fettsäureestern mittels makroporöser Kationer,

b) die Trennung des Biodiesels vom Glycerin und

c1) die Aufarbeitung des Glycerins mittels einer Reinigungseinheit aus IEC und Mischbett sowie

c2) die Aufarbeitung des Biodiesels zur Entfernung von Glycerin und/oder Seifen durch einen starksauren, monodispersen, makroporösen Kationer.

Beispielsweise können folgende Harze eingesetzt werden:

Für den Schritt a) beispielhaft Lewatit® GF 101 oder AMBERLYST® BD 20,

für den Schritt c1) beispielhaft Lewatit® GF 303 oder AMBERSEP® BD 50,

für den Schritt c2) Lewatit® K 2567 oder Lewatit® GF 202 der Lanxess Deutschland GmbH oder alternativ AMBERLITE® BD10 DRY®.

Beispiele:

Fig. 1 zeigt schematisch eine Produktionsanlage für Biodiesel mit anschließender Reinigung des Biodiesels sowie des dabei anfallenden Glycerins. Fig.2 zeigt ebenfalls schematisch eine Produktionsanlage für Biodiesel mit dem Unterschied, dass im Gegensatz zu einer Schaltung eines Mischbetts wie in Fig. 1, hier ein einziger Apparat das Mischbett enthält.

Position 1 steht in Fig. 1 für einen Apparat, der mit einem Veresterungskatalysator gefüllt ist um Fettsäuren von den Triglyceriden zu trennen. Naturgemäß bestehen natürliche Öle, beispielsweise Rapsöl, aus einem Gemisch aus Triglyceriden (>95%), Fettsäuren (0,1 bis 5 %) sowie Micellen, Phospholipiden, Proteinen und Mineralsalzen (<1%).

Bevorzugt wird in 1 ein Veresterungskatalysator vom Typ Lewatit® GF 101 oder Lewatit® K 2620 bzw. Lewatit K 2621 oder im Falle einer enzymatischen Veresterung Lewatit® GF 808 bzw. Lewatit® OC 1600 eingesetzt.

In 2 findet der Transesterifizierungsprozeß statt woran sich die Trennung der zwei Phasen, der Biodieselphase 3 von der Glycerinphase 4 anschließt.

Die Biodieselphase durchläuft einen Apparat 5 gefüllt mit beispielsweise einem monodispersen starksauren, makroporösen Kationenaustauscher vom Typ Lewatit® K 2567 oder Lewatit® GF 202 oder Lewatit® SP112 zur Entfernung von Restglycerin, Seifen, Wachse, Salzen, Wasser oder Methanol.

Die Glycerinphase durchläuft eine erfindungsgemäße Reinigungseinheit von Apparaten 6 sowie 7 und 8, bzw. alternativ 9 (Fig. 2), worin 6 für die IEC und 7 und 8 für eine Schaltung von Apparaten eines Mischbetts und 9 (Fig. 2) für einen einzelnen Apparat als Mischbett stehen. In 6 wird erfindungsgemäß ein monodisperser, gelförmiger, starksaurer Kationenaustauscher zur Abtrennung von Salzen oder Aschen aus dem Glycerin eingesetzt, beipielsweise Lewatit® GF 303.

In 7 wird beispielsweise ein monodisperser, makroporöser, starksaurer Kationenaustauscher als Polisher sowie zur Abtrennung von Kationen eingesetzt, beispielsweise Lewatit® GF 404. In 8 wird bevorzugt ein monodisperser, makroporöser, mittelbasischer Anionenaustauscher als Polisher sowie zur Abtrennung von Anionen aber auch zur Entfärbung des Glycerins eingesetzt, beispielsweise Lewatit® GF 505.

In 9 (Fig. 2) wird beispielsweise ein monodisperser, makroporöser, starksaurer Kationenaustauscher als Polisher sowie zur Abtrennung von Kationen eingesetzt, beispielsweise Lewatit® GF 404 und ein monodisperser, makroporöser, mittelbasischer Anionenaustauscher, beispielsweise Lewatit® GF 505 vom Typ-I oder Typ-II, beispielsweise Lewatit® S 6368 A oder Lewatit® S 7468 zur Abtrennung von Anionen aber auch zur Entfärbung des Glycerins in einer Mischung im Volumenverhältnis Kationenaustauscher 1 : Anionenaustauscher 0,8 - 2 eingesetzt. Die Unterscheidung von Anionenaustauschern in Typ-I oder Typ-II wird beispielsweise in Ullmanns Encyklopädie der technischen Chemie, Verlag Chemie, Weinheim, New York, 4. Aufl., Bd. 13, Seite 302 beschrieben.

Der Kationenaustauscher in Apparat 7 wird nach der Erschöpfung der vorhanden Austauscherkapazität mittels verdünnter Mineralsäuren, bevorzugt 4-10 gew.%iger Salzsäure, Schwefelsäure oder Salpetersäure regeneriert. Die Regenerierlösung kann dabei sowohl von oben als auch von unten durch das Ionenaustauscher filtriert werden. Im Anschluss wird die Regenerierlösung mit VE-Wasser (voll entsalztem Wasser) unter Beibehaltung der Filtrationsrichtung verdrängt. Danach erfolgt eine Wäsche mit VE-Wasser im Abstrom bis zu einem pH-Wert von pH 5-6 im Auslauf des Apparates.

Der Anionenaustauscher in Apparat 8 wird nach der Erschöpfung der vorhanden Austauscherkapazität mittels verdünnter Alkalilauge, bevorzugt 3-8 gew.-%iger Natronlauge regeneriert. Die Regenerierlösung kann dabei sowohl von oben als auch von unten durch den Ionenaustauscher filtriert werden. Im Anschluss wird die Regenerierlösung mit VE-Wasser unter Beibehaltung der Filtrationsrichtung verdrängt. Danach erfolgt eine Wäsche mit VE-Wasser im Abstrom bis zu einem pH-Wert von pH 7-8 im Auslauf des Apparates.

Die Komponenten der Harzmischung (Kationenaustauscher und Anionenaustauscher) im Apparat 9 (Fig. 2) werden nach Erschöpfung der Austauscherkapazität durch Rückspülen mit VE-Wasser zuerst getrennt und anschließend die separierten Harze individuell regeneriert. Dabei wird der Anionenaustauscher mit NaOH (3-6 Gew.-%) von oben und der Kationenaustauscher mit einer wässrigen Lösung von HCl, bevorzugt 5-8 gew.-%ig, von unten simultan regeneriert. Die Regenerierlösungen werden durch eine in Höhe der Harztrennzone befindliche Drainage abgezogen. Im Anschluss erfolgen die Verdrängung der Regenerierlösungen und das Auswaschen mit VE-Wasser in Richtung der jeweiligen Chemikalienlösungen.

**Tabelle 1**: Beispiel für das Auslegen einer 10.000t/Jahr IEC Anlage eines Festbetts für die Glycerin Entaschung und Entsalzung gemäß Fig. 1, Position 3 mit Lewatit® GF 303 als einzusetzendem Harz

| | |
|---|---|
| Zu reinigendes Medium | Glycerin aus der Biodiesel Transesterifizierung |
| Harzvolumen | 30 m$^3$ |
| Durchmesser des Harzbetts | 2,5 m |
| Tiefe des Harzbetts | 6,0 m |
| Beladung / Cyclus | 3,75 t Glycerin in 23 t VE-Wasser |
| Salzkonzentration des Rohglycerins | 5-7 Gew% |
| Eluat | VE-Wasser |
| Temperatur | 80 °C |
| Abfluss | 3,75 t Glycerin in 6 t VE-Wasser |
| Lebenszeit des Harzes | 5 Jahre |

VE-Wasser im Sinne der vorliegenden Erfindung ist gekennzeichnet, indem es eine Leitfähigkeit von 0,1 bis 10 µS besitzt, wobei der Gehalt gelöster oder ungelöster Metallionen nicht größer als 1 ppm, bevorzugt nicht größer als 0,5 ppm für Fe, Co, Ni, Mo, Cr, Cu als individuelle Komponenten ist und nicht größer als 10 ppm, bevorzugt nicht größer als 1 ppm für die Summe der genannten Metalle ist.

**Tabelle 2:** Beispiel für das Auslegen einer 10.000 t/Jahr Glycerin-Polyshing Mischbett Einheit

| | |
|---|---|
| Harzmenge in 7 5 m$^3$ | Lewatit® GF 404 |
| Harzmenge in 8 6 m$^3$ | Lewatit® GF 505 |
| NaCl Konzentration vor 7 | 100 ppm |
| Farbwert des Glycerins vor 7 | 200 IU |
| Durchflussrate durch 7 und 8 | 20 m$^3$/h |
| Temperatur | 60°C |
| Kapazität des Mischbetts | 3000 m$^3$/ Cyclus |
| Cycluszeit | 150 h |
| NaCl Konzentration nach 8 | < 1 ppm |
| Farbwert des Glycerins nach 8 | < 1 IU |

Die Angaben zu den Glycerin Farbwerten wurden mit einem UV/VIS Spektralphotometer Typ CADAS 30 S der Firma Dr. Lange, Berlin gemessen. Die Angaben zu den Farbwerten des Glycerins im Rahmen der vorliegenden Erfindung sind deshalb auf Messungen mit einem solchen Gerät bezogen wobei gilt:

$$IU \quad = 1000 \text{ x Ext}_{(420nm)} \text{ x } 100 \text{ / b x c x D}$$

Mit Ext = -log Transmission, b = Trockensubstanz in $^0$bx, c = Zelllänge in cm und D = Dichte.

**Herstellung von Lewatit®GF 303 für die IEC**

**Lewatit®GF 303 ist ein gelförmiger, monodisperser, stark saurer Kationenaustauscher in der Natriumform**

a) Herstellung des monodispersen, gelförmigen Perlpolymerisates

985,6 Gramm eines wässrigen Gemisches enthaltend 492,8 Gramm monodisperse mikroverkapselte Monomertröpfchen mit einer mittleren Teilchengröße von 230 µ und einem Monodispersitätsgrad von 1,11, bestehend aus 93,5 Gew. % Styrol, 6 Gew. % Divinylbenzol und 0,5 Gew. % Dibenzoylperoxid , wurden mit einer wässrigen Lösung aus 1,48 Gramm Gelatine, 2,22 Gramm Natriumhydrogenphosphatdodekahydrat und 110 mg Resorcin in 40 ml VE-Wasser in einem 4 Liter Glasreaktor versetzt.

Die Mischung wurde unter Rühren ( Rührgeschwindigkeit 220 UpM) 6 Stunden bei 70°C und anschließend 2 Stunden bei 95°C polymerisiert. Der Ansatz wurde über ein 32 µ Sieb gewaschen und getrocknet. Man erhielt 512 Gramm eines monodispersen, gelförmigen Perlpolymerisates, Perldurchmesser 275 µ, mit glatter Oberfläche.

b) Sulfonierung des monodispersen , gelförmigen Perlpolymerisates zu einem monodispersen, gelförmigen Kationenaustauscher und Überführung des Kationenaustauschers von der Wasserstoffform in die Natriumform

Apparatur : 3000 ml Doppelwandplanschliffreaktor mit Intensivkühler, Rührer und Trockenrohr

2241 g 85 gew. % ige Schwefelsäure wurden bei Raumtemperatur vorgelegt. Unter Rühren wurden in 5 Minuten 400 Gramm monodisperses, gelförmiges Perlpolymerisat dosiert. Anschließend wurden 150 ml 1,2 Dichlorethan dosiert. Die Suspension wurde 3 Stunden bei Raumtemperatur gerührt. In 1 Stunde wurden 829,8 Gramm Oleum 65 %ig dosiert. Die Suspension wurde in 1 Stunde auf 120°C erhitzt und weitere 4 Stunden bei dieser Temperatur gerührt. Dichlorethan wurde destillativ abgetrennt.

Die Suspension wurde auf Raumtemperatur abgekühlt und in eine Verdünnungsapparatur überführt wo sie mit Schwefelsäuren abnehmender Konzentration verdünnt wurde.

Das auf Raumtemperatur abgekühlte Harz wurde mit VE-Wasser gewaschen und dann klassiert.

Anschließend wurden 4400 ml 4 gew. % ige wässrige Natronlauge in 2 Stunden über das Harz filtriert und danach 3000 ml VE-Wasser über das Harz filtriert.

Ausbeute an Endprodukt : 2010 ml

Totalkapazität Menge an stark sauren Gruppen : 1,92 mol/ l

**Herstellung von Lewatit®GF 404 für das Mischbett**

**Lewatit®GF 404 ist ein makroporöser, monodisperser, stark saurer Kationenaustauscher in der Wasserstoffform**

A') Herstellung eines monodispersen, makroporösen Perlpolymerisates auf der Basis von Styrol, Divinylbenzol und Ethylstyrol

In einem 10 1 Glasreaktor wurden 3000 g voll entsalztes Wasser vorgelegt und eine Lösung aus 10 g Gelatine, 16 g di-Natriumhydrogenphosphatdodekahydrat und 0,73 g Resorcin in 320 g VE-Wasser hinzugefüllt und durchmischt. Die Mischung wurde auf 25°C temperiert. Unter Rühren wurde anschließend eine Mischung aus 3200 g mikroverkapselter Monomertröpfchen mit enger Teilchengrössenverteilung aus 8,5 Gew.-% Divinylbenzol und 2,1 Gew.-% Ethylstyrol (eingesetzt als handelsübliches Isomerengemisch aus Divinylbenzol und Ethylstyrol mit 80 % Divinylbenzol), 0,5 Gew.-% Trigonox®21 s, 56,5 Gew.-% Styrol und 32,4 Gew.-% Isododekan (technisches Isomerengemisch mit hohem Anteil an Pentamethylheptan) gegeben, wobei die Mikrokapsel aus einem mit Formaldehyd gehärteten Komplexkoazervat aus Gelatine und einem Copolymer aus Acrylamid und Acrylsäure bestand, und 3200 g wässriger Phase mit einem pH-Wert von 12 zugesetzt. Die mittlere Teilchengröße der Monomertröpfchen betrug 460 $\mu$m.

Der Ansatz wurde unter Rühren durch Temperaturerhöhung nach einem Temperaturprogramm bei 25°C beginnend und bei 95°C endend auspolymerisiert. Der Ansatz wurde abgekühlt, über ein 32 $\mu$m-Sieb gewaschen und anschließend im Vakuum bei 80°C getrocknet. Man erhielt 1893 g eines kugelförmigen , makroporösen Polymerisates mit einer mittleren Teilchengröße von 440 $\mu$m, enger Teilchengrößenverteilung und glatter Oberfläche.

Das Perlpolymerisat war in der Aufsicht kreidig weiß.

B') Sulfonierung des monodispersen, makroporösen Perlpolymerisates zu einem monodispersen , makroporösen Kationenaustauscher in der Wasserstoffform

Apparatur : 3000 ml Doppelwandplanschliffreaktor mit Intensivkühler, Rührer und Trockenrohr

1000 ml 98 gew. % ige Schwefelsäure wurden bei Raumtemperatur vorgelegt und auf 105 °C erhitzt. Unter Rühren wurden in 30 Minuten 250 Gramm monodisperses, makroporöses Perlpolymerisat dosiert. Die Suspension wurde in 1 Stunde auf 115°C erhitzt und weitere 5 Stunden bei dieser Temperatur gerührt.

Die Suspension wurde auf Raumtemperatur abgekühlt und in eine Verdünnungsapparatur überführt wo sie mit Schwefelsäuren abnehmender Konzentration verdünnt wurde.

Das auf Raumtemperatur abgekühlte Harz wurde mit VE-Wasser gewaschen und dann klassiert.

Ausbeute an Endprodukt : 1225 ml

Totalkapazität Menge an stark sauren Gruppen : 1,61 mol/ l

**Herstellung von Lewatit®GF 505 für das Mischbett**

**Lewatit®GF 505 ist ein makroporöser, monodisperser, mittel basischer Anionenaustauscher**

A") Herstellung eines monodispersen, makroporösen Perlpolymerisates auf der Basis von Styrol, Divinylbenzol und Ethylstyrol

In einem 10 1 Glasreaktor wurden 3000 g voll entsalztes Wasser vorgelegt und eine Lösung aus 10 g Gelatine, 16 g di-Natriumhydrogenphosphatdodekahydrat und 0,73 g Resorcin in 320 g VE-Wasser hinzugefüllt und durchmischt. Die Mischung wurde auf 25°C temperiert. Unter Rühren wurde anschließend eine Mischung aus 3200 g mikroverkapselter Monomertröpfchen mit enger Teilchengrössenverteilung aus 3,6 Gew.-% Divinylbenzol und 0,9 Gew.-% Ethylstyrol (eingesetzt als handelsübliches Isomerengemisch aus Divinylbenzol und Ethylstyrol mit 80 % Divinylbenzol), 0,5 Gew.-% Trigonox®21 s, 56,2 Gew.-% Styrol und 38,8 Gew.-% Isododekan (technisches Isomerengemisch mit hohem Anteil an Pentamethylheptan) gegeben, wobei die Mikrokapsel aus einem mit Formaldehyd gehärteten Komplexkoazervat aus Gelatine und einem Copolymer aus Acrylamid und Acrylsäure bestand, und 3200 g wässriger Phase mit einem pH-Wert von 12 zugesetzt. Die mittlere Teilchengröße der Monomertröpfchen betrug 460 $\mu$m.

Der Ansatz wurde unter Rühren durch Temperaturerhöhung nach einem Temperaturprogramm bei 25°C beginnend und bei 95°C endend auspolymerisiert. Der Ansatz wurde abgekühlt, über ein 32 $\mu$m-Sieb gewaschen und anschließend im Vakuum bei 80°C getrocknet. Man erhielt 1893 g eines kugelförmigen , makroporösen Polymerisates mit einer mittleren Teilchengröße von 440 μm, enger Teilchengrößenverteilung und glatter Oberfläche.

Das Perlpolymerisat war in der Aufsicht kreidig weiß und wies eine Schüttdichte von ca. 370 g/l auf.

B") Herstellung eines amidomethylierten Perlpolymerisates

Bei Raumtemperatur wurden 1856,3 ml Dichlorethan, 503,5 g Phthalimid und 351 g 29,9 gew.%iges Formalin vorgelegt. Der pH Wert der Suspension wurde mit Natronlauge auf 5,5 bis 6 eingestellt. Anschließend wurde das Wasser destillativ entfernt. Dann wurden 36,9 g Schwefelsäure zudosiert. Das entstehende Wasser wurde destillativ entfernt. Der Ansatz wurde abgekühlt. Bei 30°C wurden 134,9 g 65 %iges Oleum und anschließend 265,3 g monodisperses Perlpolymerisat, hergestellt nach Verfahrensschritt A") eindosiert. Die Suspension wurde auf 70°C erhitzt und weitere 6 Stunden bei dieser Temperatur gerührt. Die Reaktionsbrühe wurde abgezogen, VE-Wasser wurde hinzudosiert und Restmengen an Dichlorethan wurden destillativ entfernt.

Ausbeute an amidomethyliertem Perlpolymerisat : 1700 ml

Elementaranalytische Zusammensetzung:

| | |
|---|---|
| Kohlenstoff: | 75,1 Gew. %; |
| Wasserstoff: | 4,7 Gew. %; |
| Stickstoff: | 5,8 Gew. %; |
| Rest: | Sauerstoff. |

C") Herstellung eines aminomethylierten Perlpolymerisates

Zu 1680 ml amidomethyliertem Perlpolymerisat aus B") wurden 773,3 g 50 gew.-%ige Natronlauge und 1511 ml VE-Wasser bei Raumtemperatur zudosiert. Die Suspension wurde in 2 Stunden auf 180°C erhitzt und 8 Stunden bei dieser Temperatur gerührt. Das erhaltene Perlpolymerisat wurde mit VE-Wasser gewaschen.

Ausbeute an aminomethyliertem Perlpolymerisat : 1330 ml

Als Gesamtausbeute - hochgerechnet - ergeben sich 1346 ml.

Elementaranalytische Zusammensetzung:

| | |
|---|---|
| Stickstoff: | 11,6 Gew. % |
| Kohlenstoff: | 78,3 Gew. %; |
| Wasserstoff: | 8,4 Gew. %; |

Aus der elementaranalytischen Zusammensetzung des aminomethylierten Perlpolymerisates liess sich errechnen, dass im statistischen Mittel pro aromatischem Kern - herrührend aus den Styrol und Divinylbenzoleinheiten - 1,18 Wasserstoffatome durch Aminomethylgruppen substituiert wurden.

Bestimmung der Menge an basischen Gruppen : 2,17 mol/ Liter Harz

D") Herstellung eines Perlpolymerisates mit tertiären Aminogruppen

In einem Reaktor wurden 1875 ml VE-Wasser, 1250 ml aminomethyliertes Perlpolymerisat aus C") und 596,8 g 30,0 gew.-%ige Formalinlösung bei Raumtemperatur vorgelegt. Die Suspension wurde auf 40°C erwärmt. Der pH Wert der Suspension wurde durch Dosierung von 85 gew.-%iger Ameisensäure auf pH 3 eingestellt. Innerhalb von 2 Stunden wurde die Suspension auf Rückflusstemperatur (97°C) erwärmt. Während dieser Zeit wurde der pH Wert durch Dosierung von Ameisensäure bei 3,0 gehalten. Nach Erreichen der Rückflusstemperatur wurde der pH Wert zunächst durch Dosierung von Ameisensäure, dann durch Dosierung von 50 gew.-%iger Schwefelsäure auf 2 eingestellt. Es wurde 30 Minuten bei pH 2 nachgerührt. Dann wurde weiter 50 gew.-%ige Schwefelsäure hinzu dosiert und der pH Wert auf 1 eingestellt. Bei pH 1 und Rückflusstemperatur wurde weitere 8,5 Stunden gerührt.

Der Ansatz wurde abgekühlt, das Harz auf einem Sieb abfiltriert und mit VE-Wasser gewaschen.

Volumenausbeute : 2100 ml

In einer Säule wurden über das Harz 3000 ml 4 gew.-%ige wässrige Natronlauge filtriert. Anschließend wurde mit Wasser gewaschen.

Volumenausbeute : 1450 ml

Elementaranalytische Zusammensetzung :

Bestimmung der Menge an basischen Gruppen : 1,79 mol/ Liter Harz

E" ) Herstellung eines monodispersen mittelstarkbasischen Anionenaustauschers

In einem Reaktor wurden bei Raumtemperatur 700 ml Anionenaustauscher mit tertiären Aminogruppen aus Beispiel D"), 780 ml VE-Wasser und 16,5 Gramm Chlormethan vorgelegt. Der Ansatz wurde auf 40°C erwärmt und 6 Stunden lang bei dieser Temperatur gerührt.

Volumenausbeute : 951 ml

Von den stickstofftragenden Gruppen des Anionenaustauschers lagen 24,3 % als Trimethylaminomethylgruppen und 75,7 % als Dimethylaminomethylgruppen vor.

## Patentansprüche

1.  Verfahren zur Reinigung, von Glycerin mittels Ionenaustauscher, **dadurch gekennzeichnet dass** die Ionenaustauscher in einer Kombination von Elektrolytvorlaufverfahren (IEC) mit einem Mischbett eingesetzt werden und sowohl im IEC als auch im Mischbett jeweils wenigstens ein monodisperser Ionenaustauscher eingesetzt wird und diese aus monodispersen Perlpolymerisaten durch Verdüsungs- oder Saatzulaufverfahren hergestellt werden.

2.  Verfahren gemäß Anspruch 1, **dadurch gekennzeichnet, dass** im IEC starksaure Kationenaustauscher, besonders bevorzugt starksaure, gelförmige Kationenaustauscher eingesetzt werden.

3.  Verfahren gemäß einem der Ansprüche 1 oder 2, **dadurch gekennzeichnet, dass** im Mischbett ein Anionenaustauscher und ein Kationenaustauscher nebeneinander eingesetzt werden.

4.  Verwendung einer Kombination von IEC und Mischbett zur Reinigung von Glycerin, **dadurch gekennzeichnet, dass** sowohl im IEC als auch im Mischbett jeweils wenigstens ein monodisperser Ionenaustauscher eingesetzt wird und diese aus monodispersen Perlpolymerisaten durch Verdüsungs- oder Saatzulaufverfahren hergestellt werden.

5.  Verwendung gemäß Anspruch 4, **dadurch gekennzeichnet, dass** das Glycerin aus der Biodieselproduktion stammt.

6.  Verfahren zur Herstellung von Biodiesel, **dadurch gekennzeichnet, dass** der Stoffstrom des Glycerins einer Reinigungseinheit aus IEC und einem Mischbett unterzogen wird, wobei sowohl im IEC als auch im Mischbett jeweils ein monodisperser Ionenaustauscher eingesetzt wird und diese aus monodispersen Perlpolymerisaten durch Verdüsungs- oder Saatzulaufverfahren hergestellt werden.

7.  Verfahren gemäß Anspruch 6, **dadurch gekennzeichnet, dass** das Verfahren

    a) die Transesterifizierung freier Fettsäuren zu Fettsäureestern mittels makroporöser Kationer,
    b) die Trennung des Biodiesels vom Glycerin und
    c1) die Aufarbeitung des Glycerins mittels einer Reinigungseinheit aus IEC und Mischbett sowie
    c2) die Aufarbeitung des Biodiesels zur Entfernung von Glycerin und/oder Seifen durch einen starksauren, monodispersen, makroporösen Kationer umfasst.

8.  Verfahren gemäß der Ansprüche 6 oder 7, **dadurch gekennzeichnet, dass** das Mischbett in einer Schaltung von Apparaten oder in einem einzelnen Apparat vorliegt.

9.  Verfahren nach einem der Ansprüche 6 bis 8, **dadurch gekennzeichnet, dass** die Ionenaustauscher im Mischbett mit verdünnten Mineralsäuren oder Alkalilaugen regeneriert werden können.

10. Apparatur zur Biodieselherstellung enthaltend eine Reinigungseinheit aus wenigstens einem Elektrolytvorlaufverfahren (IEC) und wenigstens einem Mischbett zur Reinigung von Glycerin, **dadurch gekennzeichnet, dass** sowohl im IEC als auch im Mischbett jeweils wenigstens ein monodisperser Ionenaustauscher eingesetzt wird und diese aus monodispersen Perlpolymerisaten durch Verdüsungs- oder Saatzulaufverfahren hergestellt werden.

## Claims

1.  Method for purifying glycerol by means of ion exchangers, **characterized in that** the ion exchangers are used in a combination of ion exclusion process (IEC) with a mixed bed and at least one monodisperse ion exchanger is used in each of the IEC and the mixed bed and these are made from monodisperse bead polymers by jetting or seed/ feed processes.

2. Method according to Claim 1, **characterized in that** strong-acid cation exchangers are used in the IEC, especially preferably strong-acid, gel-like cation exchangers.

3. Method according to either of Claims 1 and 2, **characterized in that** an anion exchanger and a cation exchanger are used alongside each other in the mixed bed.

4. Use of a combination of IEC and mixed bed for purifying glycerol, **characterized in that** at least one monodisperse ion exchanger is used in each of the IEC and the mixed bed and these are made from monodisperse bead polymers by jetting or seed/feed processes.

5. Use according to Claim 4, **characterized in that** the glycerol comes from biodiesel production.

6. Method for production of biodesel, **characterized in that** the glycerol feedstock is subjected to a purification unit consisting of IEC and a mixed bed, a monodisperse ion exchanger being used in each of the IEC and the mixed bed and these being made from monodisperse bead polymers by jetting or seed/feed processes.

7. Method according to Claim 6, **characterized in that** the method comprises:

   a) the transesterification of free fatty acids into fatty acid esters by means of macroporous cation exchanger,
   b) the separation of the biodiesel from the glycerol and
   c1) the processing of the glycerol by means of a purification unit made up of IEC and mixed bed, and
   c2) the processing of the biodiesel to remove the glycerol and/or soaps by a strong-acid, monodisperse, macroporous cation exchanger.

8. Method according to Claims 6 and 7, **characterized in that** the mixed bed is present in a series of apparatus or in a single apparatus.

9. Method according to one of Claims 6 to 8, **characterized in that** the ion exchangers in the mixed bed can be regenerated with dilute mineral acids or lyes.

10. Apparatus for biodiesel production comprising a purification unit consisting of at least one ion exclusion process (IEC) and at least one mixed bed for purifying glycerol, **characterized in that** at least one monodisperse ion exchanger is used in each of the IEC and the mixed bed and these are made from monodisperse bead polymers by jetting or seed/feed processes.

**Revendications**

1. Procédé de purification de glycérine avec des échangeurs d'ions, **caractérisé en ce que** les échangeurs d'ions sont utilisés dans une combinaison d'une technique à exclusion d'ions (IEC) avec un lit mélangé, et un échangeur d'ions monodispersé est utilisé à chaque fois aussi bien dans l'IEC que dans le lit mélangé, et ceux-ci sont fabriqués à partir de polymères en perles monodispersés par des procédés d'atomisation ou d'introduction de semences.

2. Procédé selon la revendication 1, **caractérisé en ce que** des échangeurs de cations fortement acides, de manière particulièrement préférée des échangeurs de cations fortement acides en forme de gel, sont utilisés dans l'IEC.

3. Procédé selon l'une quelconque des revendications 1 ou 2, **caractérisé en ce qu'**un échangeur d'anions et un échangeur de cations sont utilisés l'un à côté de l'autre dans le lit mélangé.

4. Utilisation d'une combinaison d'une IEC et d'un lit mélangé pour la purification de glycérine, **caractérisé en ce qu'**au moins un échangeur d'ions monodispersé est utilisé à chaque fois aussi bien dans l'IEC que dans le lit mélangé, et ceux-ci sont fabriqués à partir de polymères en perles monodispersés par des procédés d'atomisation ou d'introduction de semences.

**5.** Utilisation selon la revendication 4, **caractérisée en ce que** la glycérine est issue de la production de biodiesel.

**6.** Procédé de fabrication de biodiesel, **caractérisé en ce que** le courant de matière de la glycérine est soumis à une unité de purification constituée d'une IEC et d'un lit mélangé, un échangeur d'ions monodispersé étant utilisé à chaque fois aussi bien dans l'IEC que dans le lit mélangé, et ceux-ci étant fabriqués à partir de polymères en perles monodispersés par des procédés d'atomisation ou d'introduction de semences.

**7.** Procédé selon la revendication 6, **caractérisé en ce que** le procédé comprend :

a) la transestérification d'acides gras libres en esters d'acides gras par un échangeur de cations macroporeux,
b) la séparation du biodiesel de la glycérine et
c1) le traitement de la glycérine par une unité de purification constituée d'une IEC et d'un lit mélangé, ainsi que
c2) le traitement du biodiesel pour éliminer la glycérine et/ou les savons par un échangeur de cations macroporeux monodispersé fortement acide.

**8.** Procédé selon la revendication 6 ou 7, **caractérisé en ce que** le lit mélangé est présent dans un circuit d'appareils ou dans un appareil individuel.

**9.** Procédé selon l'une quelconque des revendications 6 à 8, **caractérisé en ce que** les échangeurs d'ions dans le lit mélangé peuvent être régénérés avec des solutions d'alcalis caustiques ou des acides minéraux dilués.

**10.** Appareil pour la fabrication de biodiesel, contenant une unité de purification constituée d'au moins une technique à exclusion d'ions (IEC) et d'au moins un lit mélangé pour la purification de glycérine, **caractérisé en ce qu'**au moins un échangeur d'ions monodispersé est utilisé à chaque fois aussi bien dans l'IEC que dans le lit mélangé, et ceux-ci sont fabriqués à partir de polymères en perles monodispersés par des procédés d'atomisation ou d'introduction de semences.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7126032 B1
- EP 0411780 A
- US 2006014974 A1
- WO 8905861 A
- WO 2003056038 A1
- WO 1995017517 A1
- US 20050103622 A1

- US 5858191 A
- US 4444961 A
- EP 0046535 A
- US 4419245 A
- WO 9312167 A
- US 4382124 A

**In der Beschreibung aufgeführte Nicht-Patentliteratur**

- **Harris et al.** *Glycerol Production, Journal of Biochemical and Microbiological Technology and Engineering,* 24. September 1960, vol. II (1), 20-22